# EUROPÄISCHE PATENTANMELDUNG

(11) **EP 2 161 025 A1**
(43) Veröffentlichungstag der Anmeldung: **10.03.2010**
(21) Anmeldenummer: 08015684.7
(22) Anmeldetag: 05.09.2008
(51) Int. Cl.: A61K 36/70

(54) **Verfahren zur Herstellung eines Extraktes aus Pflanzenmaterial der Art Rumex acetosa L.**

(71) Anmelder: Engelhard Arzneimittel GmbH & Co. KG, 61138 Niederdorfelden (DE)
(72) Erfinder: Kühn, Joachim, 48157 Münster (DE); Gescher, Kirsten, 48151 Münster (DE); Hensel, Andreas, 48161 Münster (DE); Bicker, Jennifer, 20099 Hamburg (DE)
(74) Vertreter: Metten, Karl-Heinz

(57) **Zusammenfassung**

Die vorliegende Erfindung betrifft ein Verfahren zur Herstellung eines Extraktes aus Pflanzenmaterial der Art *Rumex acetosa L*., gefunden, umfassend die folgenden Schritte:
a) Zurverfügungstellung von Pflanzenmaterial der Art *Rumex acetosa* L.,
b) Zurverfügungstellung eines ersten Extraktionsmittels, enthaltend Wasser und mindestens eine zumindest in Teilen in Wasser lösliche Flüssigkeit,
c) gegebenenfalls Zurverfügungstellung eines zweiten Extraktionsmittels, enthaltend ein organisches Lösungsmittel,
d) Extraktionsbehandlung des Pflanzenmaterials mit dem ersten Extraktionsmittel unter Erhalt eines Extraktes,
e) Abtrennen des Extraktes von dem extrahierten Pflanzenmaterial,
f) gegebenenfalls Einengung, z.B. Trocknung, des abgetrennten Extraktes, und
g) gegebenenfalls Aufreinigung und/oder Aufkonzentrierung des Extraktes,

Ferner betrifft die vorliegende Erfindung einen Extrakt zur Herstellung eines Arzneimittels, insbesondere zur Behandlung von Virusinfektionen.

## Beschreibung

### Verfahren zur Herstellung eines Extraktes aus Pflanzemnaterial der Art Rumex acetosa L

Die vorliegende Erfindung betrifft ein Verfahren zur Herstellung eines Extraktes auf Pflanzenmaterial der Art *Rumex acetosa L.,* die nach diesem Verfahren erhältlichen Extrakte sowie deren Verwendung bei der Behandlung viraler Infektionen.

In der Arzneimittelkunde sind die heilsamen Wirkungen des Sauerampferkrauts ("Rumices Herba") seit langem bekannt. Mittlerweile werden unterschiedlichste Rumex-Arten für jeweils sehr spezielle Anwendungen eingesetzt. Auch kommen Mischungen an Rumex-Arten zum Einsatz. Beispielsweise offenbart die DE 10 2005 053 926 B3 die Verwendung eines antibakteriellen Mittels aus einer Mischung aus Pflanzendrogen, bestehend aus *Rumex acetosa L*., Rumex acetosella L., Rumex obtusifolius L., Rumex patientia L., Rumex crispus L., Verbena officinalis, Sambucus nigra, Primula veri und Gentiana lutea.

Ferner ist aus der JP 10 2003 0023 111 A bekannt, Methylenchlorid-Extrakte von *Rumex acetosa L.* zur Behandlung von Krebsarten wie Lungenkrebs, Eierstockkrebs oder Hautkrebs einsetzen zu können. Gemäß der JP 10 2005 00 20 950 A soll sich aus einem *Rumex acetosa L.-*Extrakt die Wirkverbindung Emodin erhalten lassen. Hierfür ist zunächst getrocknetes Rumex acetosa L.-Pflanzenmaterial in die Pulverform zu überführen. Das Pulver wird mit einer Mischung an Methanol und Wasser aufgenommen und in der Weise erhitzt, dass man einen Wasserextrakt von *Rumex acetosa L.* erhält. Durch Zugabe von Wasser und Hexan gelangt man unter Rühren zu einem Zweiphasengemisch. Die wässrige Phase wird mit Methylenchlorid behandelt. Die Methylenchloridphase wird abgetrennt, auf ein geringes Volumen eingeengt und anschließend chromatographisch unter Verwendung einer Mischung aus Methylenchlorid und Methanol als Eluens isoliert. Der erhaltene Wirkstoff Emodin soll als Antikrebsmittel dienen.

Aus der JP 10 063 80 53 B1 ist bekannt, einen Extrakt von *Rumex crispus* kosmetischen Zusammensetzungen beizufügen, um Hautreizungen zu vermeiden.

In der JP 10 2005 0043 217 A wird ein Extrakt von Rumex acetosella L. offenbart, welcher über eine gesteigerte herbizide Wirkung verfügen soll. Hierfür ist eine trockene Probe von Rumex acetosella L. mit Methanol zu behandeln. Aus diesem Methanol-Extrakt kann durch Versetzen mit Hexan das herbizide Prinzip abgetrennt und mittels Säulenchromatographie aufgereinigt werden. Bei dem wirkprinzip soll es sich um Chrysophansäure handeln,

Der WO 03/099307 A1 entnimmt man ein Verfahren zur Isolierung von Quercetin-3-O-β-D-glucuronid aus einem Extrakt von Blättern der Pflanzenart Rumex aquaticus herba. Diese Verbindung soll zur Behandlung von z.B. Gastritis geeignet sein. Aus der JP 10 2007 0096 279 A geht die Verwendung eines Extrakt von Rumex crispus L. für kosmetische Zusammensetzungen hervor, In der JP 10 2006 0078 150 A wird eine pharmazeutische Zusammensetzung zur Behandlungen von Diabeteserkrankungen beschrieben, welche auf dem kombinierten Einsatz eines Extraktes von Rumex japonicus und Rumex crispus basiert. Der JP 10 2006 0106 554 A entnimmt der Fachmann den antibakteriellen Einsatz eines Extraktes von Rumex acetosella L. bei der Behandlung von Staphylococcus aureus.

Als geeignete Extrakte zur Behandlung von Herpesviren nennt die DE 10 2006 015 573 A1 einen Extrakt eines Rhabarberwurzelmaterials. Hierbei ist eine getrocknete und gemahlene Wurzelprobe mehrmals einer Wirbelextraktion mit Methanol im Ultraschallbad zu unterwerfen. Dieses Verfahren ist äußerst aufwendig und lässt hinsichtlich des Wirkungsspektrums des erhaltenen Extraktmaterials noch Wünsche offen.

Der vorliegenden Erfindung lag die Aufgabe zugrunde, ein Mittel verfügbar zu machen, dass mit geeigneter antiviraler Aktivität, z.B. gegen Adeno- und/oder Herpes Simples-Viren, aus-gestattet und gleichwohl auf einfache und zuverlässige Art und Weise mit kostengünstigen und umweltschonenden Mitteln zugänglich ist Des weiteren lag der vorliegenden Erfindung die Aufgabe zugrunde, ein Behandlungsmittel verfügbar zu machen, dass einerseits mit einer guten antiviralen Aktivität ausgestattet ist und andererseits über keine bzw. nur eine sehr geringe Zelltoxizität verfügt.

Demgemäß wurde ein Verfahren zur Herstellung eines Extraktes aus Pflanzenmaterial der Art *Rumex acetosa L.,* gefunden, umfassend die folgenden Schritte:
a) Zurverfügungstellung von Pflanzenmaterial der Art Rumex *acetosa L.,*
b) Zurverfügungstellung eines ersten Extraktionsmittels, enthaltend Wasser und mindestens eine zumindest in Teilen in Wasser lösliche Flüssigkeit,
c) gegebenenfalls Zurverfügungstellung eines zweiten Extraktionsmittels, enthaltend ein organisches Lösungsmittel,
d) Extraktionsbehandlung des Pflanzenmaterials mit dem ersten Extraktionsmittel unter Erhalt eines Extraktes,
e) Abtrennen des Extraktes von dem extrahierten Pflanzenmaterial,
f) gegebenenfalls Einengung, z.B. Trocknung, des abgetrennten Extraktes, und
g) gegebenenfalls Aufreinigung und/oder Aufkonzentrierung des Extraktes,

Bevorzugt wird als Pflanzenmaterial das Kraut von *Rumex acetosa L.* eingesetzt, welches vorzugsweise Blätter und Stängel umfasst. Dieses Pflanzenmaterial wird bevorzugt in getrocknetem Zustand der Extraktionsbehandlung unterworfen. Bevorzugt ist ferner, das Pflanzenmaterial in zerkleinerter Form zur Verfügung zu stellen. Hierbei hat es sich in einer Ausführungsform als zweckmäßig erwiesen, dass das Pflanzenmaterial eine durchschnittliche maximal Ausdehnung bis etwa 10 mm, vorzugsweise bis etwa 6 mm, aufweist. Beispielsweise können die Pflanzenbestandteile auf eine Größe kleiner 5 x 5 mm und insbesondere kleiner 2 x 2 mm zerkleinert werden. Bevorzugt wird das Pflanzenmaterial in Pulverform für die Extraktion eingesetzt. Hierbei können z.B. vorzugsweise mehr als 50 Gew.-% des Pflanzenmaterials in Pulverform, insbesondere mehr als 80 Gew.-%, vorliegen. In einer besonders zweckmäßigen Ausgestaltung liegt das gesamte Pflanzenmaterial, das bei der Extraktion zum Einsatz kommt in, vorzugsweise trockener, Pulverform vor. Selbstverständlich kann in einer zweckmäßigen Variante das zerkleinerte Pflanzenmaterial auch eine durchschnittliche maximale Ausdehnung im Bereich von 5 bis 8 mm aufweisen.

Bei der in Wasser zumindest in Teilen löslichen Flüssigkeit als Bestandteil des ersten Extraktionsmittels wird vorzugsweise auf Alkohole oder Aceton oder Mischungen hiervon zurückgegriffen. Gemäß einer weiteren Ausgestaltung kann vorzugsweise vorgesehen sein, dass das erste Extraktionsmittel ein Alkohol/Wasser-Gemisch, insbesondere ein Ethanol/WasserGemisch, oder ein Aceton/Wasser-Gemisch darstellt. Des weiteren können Mischungssysteme mit mehr als zwei Komponenten als Extraktionsmittel eingesetzt werden, z.B. ternäre Systeme. Insbesondere bevorzugt ist, wenn als Alkohol ein C₂₋₅-Alkohol, beispielsweise Ethanol, iso- oder n-Propanol oder n-, iso- oder t-Butanol eingesetzt werden. Ethanol ist besonders bevorzugt. Selbstverständlich können auch Mischungen an Alkoholen als Alkoholkomponente des Extraktionsmittels verwendet werden. Dabei kann in einer besonders zweckmäßigen Ausgestaltung vorgesehen sein, dass in dem Alkohol/Wasser-Gemisch, insbesondere dem Ethmol/Wasser-Gemisch, der Alkoholanteil im Bereich von 20 bis 80 Vol.-%, insbesondere im Bereich von 40 bis 60 Vol.%, liegt. Zufriedenstellende Resultate stellen sich insbesondere auch dann ein, wenn das Alkohol/Wasser-Gemisch, insbesondere das Ethanol/Wasser-Gemisch im wesentlichen etwa 50 Volumenteile Ethanol und im wesentlichen etwa 50 Volumenteile Wasser enthält.

Alternativ kann z.B. auf Aceton/Wasser-Gemische als Extraktionsmittel zurückgegriffen werden. Hierbei ist z.B. bevorzugt, wenn der Aceton-Anteil im Aceton/Wasser-Gemisch im Bereich von 50 bis 90 Vol.-%, insbesondere im Bereich von 60 bis 80 Vol.-%, liegt.

Das Gewichtsverhältnis von zu extrahierendem Pflanzenmaterial zu Extraktionsmittel liegt bei dem erfindungsgemäßen Verfahren in einer Ausführungsform üblicherweise im Bereich von 1:1 bis 1:20, insbesondere im Bereich von 1:1 1 bis 1:10.

Die Extraktionsbehandlung gemäß Schritt d) kann z.B. bei Raumtemperatur vorgenommen werden. Alternativ kann vorgesehen sein, die Extraktion bei Temperaturen unterhalb Raumtemperatur oder bei Temperaturen oberhalb Raumtemperatur vorzunehmen. Die hierfür zu verwendenden Kühl- bzw. Heizaggregate sind dem Fachmann bekannt. Je nach eingesetzter Menge an Pflanzenmaterial kann die Dauer der Extraktionsbehandlung in weiten Bereichen variieren. Üblicherweise ist es ausreichend, die Extraktionsbehandlung gemäß Schritt d) über einen Zeitraum von bis zu 48 h vorzunehmen. Im allgemeinen reicht auch häufig ein Zeitraum von 12 h.

Ganz allgemein kann für die Extraktion gemäß dem erfindungsgemäßen Verfahren auf alle dem Fachmann geläufigen Extraktionsverfahren, wie die Mazeration, Dimazeration, Wirbelextraktion, Perkolation, Soxhletverfahren, Ultra-turrax-Extraktion, Ultraschallextraktion, Gegenstromextraktion oder Heißextraktion zurückgegriffen werden.

In einer pragmatischen Ausgestaltung kann das Extrakt von dem extrahierten Pflanzenmaterial mittels Dekantierens getrennt werden. Alternativ oder zusätzlich kann die Abtrennung des Extraktes durch Filtration und/oder Zentrifugation erfolgen. Geeignete Zentrifugen und Filtriermittel sind dem Fachmann bekannt. Für eine weitere Aufreinigung des Extraktes kann dieser chromatographischen Trennverfahren unterworfen werden, z.B. der Adsorptionschromatographie und/oder der Verteilungschromatographie. Häufig reicht es auch bereits aus, den abgetrennten Extrakt mit geläufigen Verfahren einzuengen, um z.B. zu der Trockensubstanz oder zu einem hochkonzentrierten Extrakt zu gelangen.

Gemäß einem weiteren Aspekt des erfindungsgemäßen Verfahren ist es möglich, das in den Schritten d), e), f) und/oder g) erhaltene Extrakt, z.B. in Form eines eingeengten Extraktes oder als Trockenextrakt, mit einem zweiten Extraktionsmittel, vorzugsweise in Gegenwart von Wasser, zu behandeln. Hierdurch können Extraktvarianten erhalten werden, deren Zusammensetzungen, insbesondere hinsichtlich der unwirksamen Begleitstoffe, geringfügig variieren. Beispielsweise kann als zweites Extraktionsmittel auf ein unpolares organisches Lösungsmittel, insbesondere ein Kohlenwasserstoff oder einen Kohlenwasserstoffgemisch, z.B. Benzin oder Petrolether, und/oder Ethylacetat zurückgegriffen werden. Besonders bevorzugt wird der gemäß Schritt d) erhaltene Extrakt, z.B. in vollständig oder partiell eingeengter Form, mit dem zweiten Extraktionsmittel, z.B. Petrolether, in Gegenwart von Wasser behandelt. Hierbei ist es möglich, Wasser zu dem nach Schritt d) erhaltenen Extrakt hinzuzusetzen, auch wenn in dem Extrakt selber noch Wasser vorliegt, oder ohne Wasserzusatz auszukommen, insbesondere wenn der nach Schritt d) erhaltene Extrakt selber Wasser enthält, Wird kein Wasser extern zugegeben, spricht man häufig auch von Ausschütteln. Die mit dem zweiten Extraktionsmittel nach der Extraktion bzw. dem Ausschütteln erhaltene Phase wird regelmäßig verworfen. Darin können z.B. Bestandteile enthalten sein wie Chlorophyll, welche man aus dem Extrakt entfernen möchte,

Zur Aufkonzentrierung bzw. Einengung des Extraktes kann auf dem Fachmann bekannte Methoden wie die Trocknung, die Vakuumbandtrocknung, Sprühtrocknung oder die Gefriertrocknung, auch Lyophilisierung genannt, zurückgegriffen werden. Unter Einengung im Sinne der vorliegenden Erfindung soll u.a. verstanden werden, dass das Extrakt bis zur Trockne eingeengt, d.h. von z.B. Lösungsmittel und/oder wässrigen Anteilen befreit wird. Ein solcher Extrakt wird regelmäßig auch als Trockenextrakt bezeichnet. Des Weiteren sollen auch solche eingeengten Extrakte umfasst sein, bei denen Lösungsmittel bzw. wässrige Anteile nicht vollständig entfernt worden sind. Solche Extrakte verfügen regelmäßig über eine zähflüssige, z.B. sirupöse Konsistenz und sind auch als so genannte Dickextrakte bekannt, In einer Ausführungsform ist es nicht erforderlich, den Extrakt bis zum Dickextrakt einzuengen. Die Einengung bis zur Trockne, d.h. der Erhalt eines Trockenextrakts ist im vorliegenden Fall bevorzugt.

Die nach dem erfindungsgemäßen Verfahren erhältlichen Extrakte verfügen über eine ausgesprochen gute antivirale Aktivität, beispielsweise gegenüber Adeno-, Herpes Simplex-, Rhino-, Influenza A-, Influenza B-, Parainfluenza-, Corona-Viren oder mit dem Respiratory syncytial- oder dem Humanes Metapneumovirus, und können z.B. in kosmetischen Produkten, Medizinprodukten, Arzneimitteln und Nahrungsergänzungsmitteln eingesetzt werden. Die Aktivität gegenüber Adeno- und/oder Herpes Simplex-Viren sei hervorgehoben.

Die Erfindung betrifft somit ferner die Verwendung des erfindungsgemäß hergestellten Extraktes, insbesondere des Trockenextraktes, zur Herstellung eines Medizinproduktes oder Arzneimittels, insbesondere eines Arzneimittels, zur Behandlung von Virusinfektionen, beispielsweise von Infektionen mit Rhino-, Influenza A-, Influenza B-, Parainfluenza-, Corona-Viren oder mit dem Respiratory syncytial- oder dem Humanes Metapneumovirus, insbesondere mit dem Herpes Simplex-Virus oder dem Adeno-Virus. Ferner betrifft die Erfindung die Verwendung des erfindungsgemäß hergestellten Extraktes, insbesondere des Trockenextraktes, zur Herstellung von kosmetischen Präparaten und Nahrungsergänzungsmitteln.

Die das nach dem erfindungsgemäßen Verfahren erhältliche Extrakt enthaltende Formulierung, z.B. in Form eines Arzneimittels, Medizinproduktes, kosmetischen Produkts oder Nahrungsergänzungsmittels, kann u.a. in Form von Kapseln, Tabletten, Dragees, Pastillen, Sprays, Schaum, Zäpfchen, Granulat, Pulver, Lösungen, Cremes, Emulsionen, Aerosolen, z.B. als pulmonales Inhalat, Salben, Ölen, Gels, wässrigen Extrakten, Transferosomen oder enkapsuliert in Liposomen, Mizellen oder Mikrosphären vorliegen. Des weiteren kann der nach dem erfindungsgemäßem Verfahren erhältliche Extrakt in Pflaster und Implantate eingearbeitet sein bzw. auf diesen vorliegen.

Mit der vorliegenden Erfindung geht die überraschende Erkenntnis einher, dass sich die nach dem erfindungsgemäßen Verfahren erhältlichen Extrakte von Pflanzenmaterial der Art *Rumex acetosa L.* durch eine überaus ausgeprägte antivirale Wirkung, kombiniert mit einer sehr geringen oder nicht vorhandenen Zytotoxizität auszeichnen.

Weitere Vorteile der vorliegenden Erfindung ergeben sich aus der nachfolgenden Beschreibung von Beispielen und den beigefügten Figuren. Hierin zeigen
- Figur 1: die in Diagrammform dargestellte antivirale Aktivität des Rumex-Extraktes 1 gegenüber Herpes Simplex-Viren (HSV-1) auf Vero-Zellen,
- Figur 2: die in Diagrammform dargestellte antivirale Aktivität des Rumex-Extraktes 2 gegenüber Herpes Simplex-Viren (HSV-1) auf Vero-Zellen, und
- Figur 3: Immunfluoreszenz-Mikroskopieaufnahmen in a) 400-, b) 630- und c) 1000-facher Vergrößerung.

### Beispiele:

1) Blattmaterial von *Rumex acetosa L.* wurde getrocknet und anschließend zerkleinert und pulverisiert. 25 g dieses getrockneten Pulvermaterials wurden in einem ersten Versuch mit 150 ml eines 1:1- Ethanol/Wasser-Gemisches behandelt, und zwar bei 90°C für 30 min und anschließend für 20 h bei Raumtemperatur (Extrakt Rumex 2). In einem zweiten Versuch wurde die gleiche Menge an pulverförmigem trockenen Blattmaterial von *Rumex acetosa L.* mit 250 ml eines 7:3 Aceton/Wasser-Gemisches unter Erhalt des Extraktes Rumex 1 behandelt, und zwar für einen Zeitraum von 2 x 10 min bei einer Temperatur von 25°C. Die erhaltenen Extrakte wurden mittels Filtrierens von dem extrahierten Pflanzenmaterial getrennt und mit Hilfe der Gefriertrocknung zur Trockne gebracht.
2) Für die Versuche zum Nachweis der antiviralen Wirkung wurden Herpesviren (Laborstamm 17syn+, im weiteren HSV-1 genannt) und Vero-Zellen (Meerkatzen-Nierenzellen von *Cercopithecus aethiops*) verwendet. Alle Tests wurden mit Medium ohne Zusatz von fetalem Kälberserum (FCS) durchgeführt.
   Es wurden die folgenden Kulturmedien eingesetzt:
   10% FCS zur Vorkultivierung und Zellstammhaltung
   MEM-Earle w 2,2 g/I NaHCO₃ (500 ml Gebinde)
   + 5 ml NEA (Non-essential amino acids; Biochrom AG, Berlin, Kat-Nr. K0293)
   + 5 ml L-Glutamine (Sigma, Steinheim, Kat.-Nr. G7513)
   + 5 ml Antibotic-Antimycotic (Gibco/Invitrogen Cooperation, Kat,-Nr. 15240-096)
   + 50 ml FCS

   w/o FCS für Tests auf antivirale Aktivität:
   MEM-Earle w 2,2 g/l NaHCO₃ (500 ml Gebinde)
   + 5 ml NEA (Non-essential amino acids; Biochrom AG, Berlin, Kat.-Nr. K0293)
   + 5 ml L-Glutamine (Sigma, Steinheim, Kat.-Nr. G7513)
   + 5 ml Antibotic-Antimycotic (Gibco/Invitrogen Cooperation, Kat.-Nr. 15240-096)
   Für die Auswertung wurden die folgenden Parameter herangezogen bzw. untersucht:
   CC₅₀ = Konzentration, der eine Zelltoxizität von 50% zugeordnet wird. IC₅₀ = minimale Konzentration, die 50 % antivirale Aktivität aufweist; S1 = Selektivitätsindex = CC₅₀/IC₅₀.
   Die nachfolgend verwendeten Methoden finden sich im übrigen auch beschrieben bei:
   Cheng, H.Y., Lin, C. C., & Lin, T. C. (2002). Antiherpes simplex virus type 2 activity of casuarinin from the bark of Terminalia arjuna linn. Antiviral Research, 55(3), 447 - 455; Cheng, H. Y., Lin, T. C., Yang, C. M., Wang, K. C., Lin, L. T., & Lin, C. C. (2004). Putranjivain A from Euphorbia jolkini inhibits both virus entry and late stage replication of herpes simplex virus type 2 in vitro. The Journal of antimicrobial chemotherapy, 53(4), 577 - 583; und De Logu, A., Loy, G., Pellerano, M. L., Bonsignore, L., & Schivbo, M. L. (2000), Inactivation of HSV-1 and HSV-2 and prevention of cell-to-cell virus spread by Santolina insularis essential oil. Antiviral Research, 48(3), 177 - 185.

3) Plaques Reduction Assay (Direct Plaque Assay)
   a) 1,25 x 10⁵ Zellen wurden pro Vertiefung einer 24-well Zellkulturplatte ausgesät und bis zur Konfluenz vorkultiviert. Das Virus wurde Testextrakten in Konzentrationen von 100 bis 0,01 µg/ml für 1h bei 37°C vorinkubiert. Der Zellmonolayer wurde dann mit 100 PFU dieser vorbehandelten HSV-1 infiziert. Als Kontrolle wurden unbehandelte Viren zu den Zellen gegeben. Nach einstündiger Inkubation wurde der Überstand von den Zellen entfernt, zweimal mit PBS gewaschen und Überschichtungs-Medium, ein 1:1 Mix aus 2-fach Modified Eagle Medium und 2 % Carboxymethylcellulose (CMC), auf die Zellen gegeben. CMC inaktiviert frei vorliegende Viruspartikel, so dass eine Ausbreitung der Viren nur in direkt angrenzende Nachbarzellen möglich war. Drei Tage später wurden die Zellen mit 3,7 %iger Fomaldehyd-Lösung fixiert und mit 1%iger Kristallviolett-Lösung gefärbt. Durch Auszählen der Plaques wurde die antivirale Aktivität ermittelt.
   b) Alternativ wurde der Versuch wie unter a) beschrieben wiederholt, mit dem Unterschied, dass die Substanz erst zum Überschichtungsmedium hinzugegeben wurde.
4) MTT-Assay
   a) Vero-Zellen wurden in 96-well Zellkulturplatten ausgesät (1 x 10⁴ Zellen pro Vertiefung) und für 24 h kultiviert. Die Viren wurden 1h mit Extrakt, erhalten gemäß Ziffer 1) in Konzentrationen von 100 bis 0,01 µg/ml vorinkubiert und anschließend auf den Zellmonolayer gegeben (moi = 1). Die Zugabe von unbehandelten Viren diente als Kontrolle. Nach 48 h wurde der Überstand von den Zellen entfernt, die Zellen zweimal mit PBS gewaschen, das MTT-Reagenz zugegeben und für 4h inkubiert. Die gebildeten Formazankristalle wurden in DMSO gelöst und die Adsorption bei 492 nm gegen eine Referenzwellenlänge von 650 nm vermessen.

   b) Zellen wurden mit HSV-1 (moi = 1) infiziert. Nach einstündiger Inkubation wurden die Testsubstanzen in Konzentrationen 100 bis 0,01 µg/ml zugegeben.
   c) Zellen wurden mit HSV-1 (moi = 1) infiziert. Nach einstündiger Inkubation wurde der Überstand abgenommen, die Zellen wurden zweimal mit PBS gewaschen und in frischem Medium gelöste Testsubstanzen in Konzentrationen 100 bis 0,01 µg/ml zugegeben. Der Überstand wurde zusätzlich abgenommen und wie unter Punkt 6) beschriebenen austitriert, um zu überprüfen, ob im Überstand infektiöse Partikel vorhanden sind.
   d) Es wurde wie unter Ziffer a) beschrieben vorgegangen, jedoch wurde nach 1h Inkubation von Virus, Extrakt und Zellen der Überstand entfernt, die Zellen wurden zweimal mit PBS gewaschen und frisches Medium zugegeben.
5) Zytotoxizitäts-Assay
Zur Überprüfung eines eventuell zytotoxischen Effekts der Testsubstanzen auf die Zellen wurde der MTT-Assay verwendet. Dieser wurde wie der unter Punkt 4)a) beschriebene Assay, aber ohne Zugabe von Virus durchgerührt.
6) Plaque-Assay (Titration des Überstands aus 4)c)
Vero-Zellen wurden in Zellkulturplatten mit 6 bzw. 24 Vertiefungen in 10% FCS-Medium ausgesät. Pro Vertiefung einer 6-Well-Platte wurden ca. 6 x 10⁵ Zellen, je 24er Vertiefung 1,25 x 14⁵ eingesetzt. Nach eintägiger Kultivierung und Bildung eines konfluenten Zellrasens wurden Verdünnungen des Überstandes (von 10⁻¹ bis 10⁻⁶) auf die Zellen gegeben. Nach einer Stunde Inkubation bei 37°C wurde die Virussuspension von den Zellen abgenommen, die Zellen mit PBS gewaschen und Überschichtungsmedium (1% Carboxymethylcellulose, s. 3)a)) zugegeben. 3 Tage später wurden die Zellen mit 3,7%iger Formaldehyd-Lösung fixiert und mit 1%iger Kristallviolett-Lösung gefärbt. Nach Auszählen der Plaques wurde die antivirale Aktivität ermittelt.
7) Plaque-Assay (Vorinkubation Virus und Substanz, Substanz während Adsorption und Penetration)
Virus und Substanz wurden 24 h bei 37°C vorinkubiert und anschließend wie unter Punkt 6) beschrieben austitriert. Wie bei den Versuchen aus 3)a) und 4)d) konnte auf einen Einfluss der Testextrakte direkt auf das Virus (Vorinkubation) oder während der Adsorptions- und Penetrationsphase geschlossen werden.

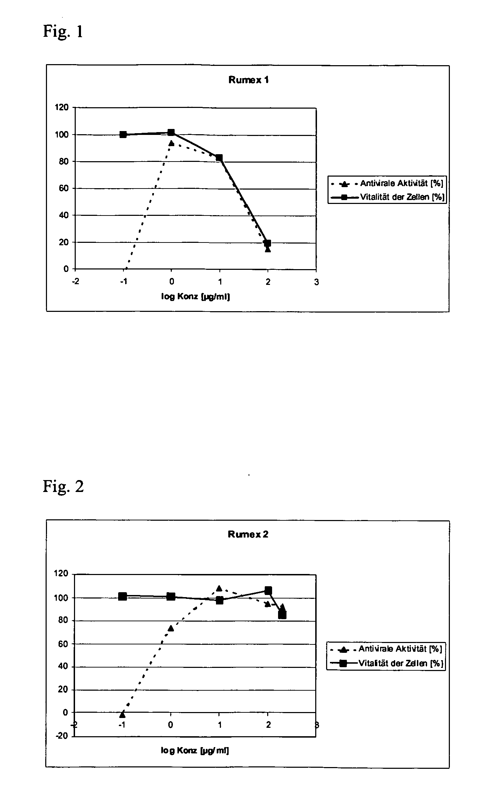

Figuren 1 und 2 zeigen die im MTT-Assay gemäß Ziffer 4)a) erzielten Effekte auf Vero-Zellen bezüglich Infektiösität und Vermehrung von HSV-1. Figur 2 ist zu entnehmen, dass der Extrakt Rumex 2 im Konzentrationsbereich 1 µg/ml eine sehr starke antivirale Aktivität bei abwesender Zelltoxizität aufweist. Der Extrakt Rumex 2 zeigt gemäß Figur 2 eine gute antivirale Aktivität bei 1 µg/ml sowie eine maximale Wirkung bei Konzentration zwischen 10 und 100 µg/ml. Gleichzeitig konnte bei allen getesteten Konzentrationen bis 200 µg/ml keine signifikante Zelltoxizität verzeichnet werden. Für den Extrakt Rumex 2 ergab sich ein Selektivitätsindex SI von ca. 700 (SI = CC₅₀/IC₅₀). Wie Figur 1 zeigt, hat Extrakt Rumex 1 ebenfalls zwischen 1 und 10 µg/ml deutlich antivirale Effekte, gleichzeitig ist auch bei diesem Konzentrationsbereich eine Schädigung der Verumzellen zu verzeichnen. Die antivirale Wirkung der Extrakte Rumex 1 und Rumex 2 konnte auch durch den Plaque-Reduction Test gemäß Ziffer 3) bestätigt werden. Der nachfolgenden Tabelle entnimmt man die antivirale Wirkung der Extrakte Rumex 1 und Rumex 2 in verschiedenen Konzentrationen gegenüber HSV-1 und Vero-Zellen im Plaque-Reduction-Assay:

| | | | | | |
|---|---|---|---|---|---|
| | 100 µg/ml | 10 µg/ml | 1 µg/ml | 0,1 µg/ml | 0,01 µg/ml |
| Rumex 1 | 100+/-0 | 100+/-0 | 100+/-0 | 100+/-0 | 98+/-0 |
| Rumex 2 | 100+/-0 | 100+/-0 | 100+/-0 | 89+/-2 | -23+/-15 |

Im Zusammenhang mit dem vorgenommenen Test wurde festgestellt, dass sich die Inaktivierung der mit den Extrakten behandelten Viruspartikel durch nachfolgende Wasch- oder Verdünnungsschritte nicht aufheben lässt.

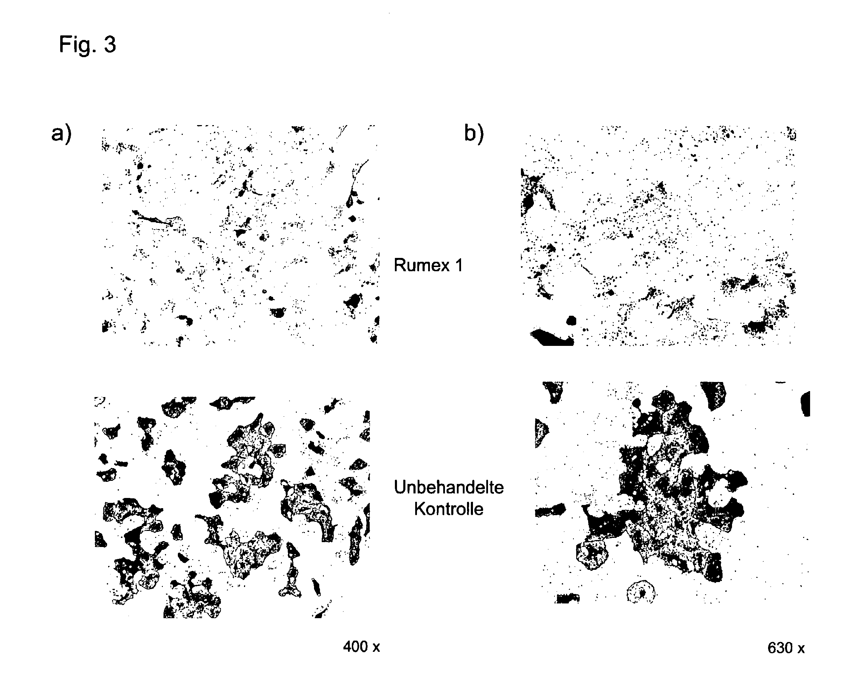

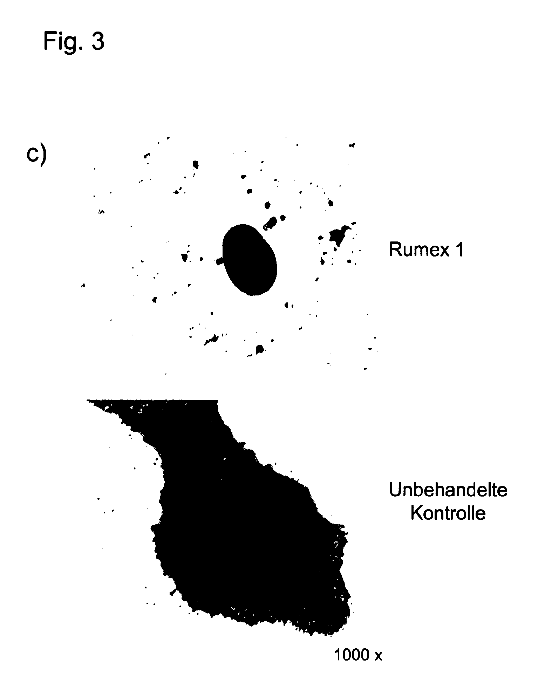

Figur 3 zeigt Immunfluoreszenz-Mikroskopieaufnahmen von Assays mit HSV-1 infizierten Vero-Zellen, welche in einer ersten Ausführungsform mit dem Extrakt Rumex 1 behandelt wurden und welche zu Vergleichszwecken in einer weiteren Ausführungsform nicht mit einem solchen Extrakt behandelt wurden, in a) 400-, b) 630- und c) 1000-facher Vergrößerung. Hierzu wurden zunächst Vero-Zellen in 24-Well-Platten (10⁵ Zellen/Well) ausgesät und bis zur Konfluenz von ca. 50 % für eine Dauer von etwa 24 Stunden kultiviert. Das Virus (HSV-1) sowie der Extrakt Rumex 1 wurden für eine Stunde bei 37°C inkubiert. Die Vero-Zellen, das Virus und der Extrakt wurden auf 4°C heruntergekühlt. Das Virus (100 PMU/Well) und der Extrakt wurden zu den Vero-Zellen gegeben und 20 min bei 4°C gehalten. Anschließend wurde der Überstand abgenommen, mit PBS gewaschen, um nicht absorbierte Viren zu entfernen. Die Zellen wurden bei -20°C durch Zugabe von Methanol über einen Zeitraum von 30 min fixiert. Nach dieser Fixierung wurde eine Blocklösung (2% BSA in PBS) für 20 min zugegeben, um unspezifische Bindungen zu verhindern. Anschließend wurde ein erster Antikörper (Rabbit Anti HSV-1, Dako, Denmark, Code-Nr. B0114) über einen Zeitraum von einer Stunde zugegeben. Nach dreimaligem Waschen mit BSA/PBS und Zugabe eines zweiten Antikörpers (Biotin-SP-conjugated AffiniPure F(ab')₂ Fragment Goat-Anti-Rabbit IgG, Dianova, Hamburg, Code-Nr. 111-066-045) wurde wiederum über den Zeitraum von einer Stunde inkubiert. Nach einem weiteren Waschschritt wurde zur Detektion der Antigen-Antikörper-Bindung ein Fluoreszenzfarbstoff (Cy^{™}3-conjugated Streptavidin, Dianova, Hamburg, Code-Nr. 016-160-084) zugegeben. Gleichzeitig erfolgte die Färbung der Zellkerne mittels DAPI.

Die spezifische Anfärbung von HSV-1 mittels Immunfluoreszenzfärbung zeigte, dass in den unbehandelten Kontrollversuchen starke Adsorption der Viren an die Wirtszelle erfolgt (siehe in Fig. 3: Kontrolle unbehandelt). Die Vorbehandlung der Viren mit dem Extrakt Rumex 1 und die nachfolgende Behandlung der Vero-Zellen dieser viralen Präparationen führten zu einer deutlich verringerten Adsorption der Viren an die Zellen (siehe in Fig. 3: Rumex 1).

Die in der vorstehenden Beschreibung, in den Ansprüchen sowie in den Zeichnungen offenbarten Merkmale der Erfindung können sowohl einzeln aus auch in jeder beliebigen Kombination für die Verwirklichung der Erfindung in ihren verschiedenen Ausfuhrungsformen wesentlich sein.

## Patentansprüche

1. Verfahren zur Herstellung eines Extraktes aus Pflanzenmaterial der Art *Rumex acetosa* L., umfassend die folgenden Schritte:
a) Zurverfügungstellung von Pflanzenmaterial der Art *Rumex acetosa* L.,
b) Zurverfügungstellung eines ersten Extraktionsmittels, enthaltend Wasser und mindestens eine zumindest in Teilen in Wasser lösliche Flüssigkeit,
c) gegebenenfalls Zurverfügungstellung eines zweiten Extraktionsmittels, enthaltend ein organisches Lösungsmittel,
d) Extraktionsbehandlung des Pflanzenmaterials mit dem ersten Extraktionsmittel unter Erhalt eines Extraktes,
e) Abtrennen des Extraktes von dem extrahierten Pflanzenmaterial,
f) gegebenenfalls Einengung des abgetrennten Extraktes, und
g) gegebenenfalls Aufreinigung und/oder Aufkonzentrierung des Extraktes.

2. Verfahren nach Anspruch 1, **dadurch gekennzeichnet, dass** es sich bei dem Pflanzenmaterial um getrocknetes Pflanzenmaterial handelt.

3. Verfahren nach Anspruch 1 oder 2, **dadurch gekennzeichnet, dass** das Pflanzenmaterial in zerkleinerter Form zur Verfügung gestellt wird.

4. Verfahren nach Anspruch 3, **dadurch gekennzeichnet, dass** das, insbesondere zerkleinerte, Pflanzenmaterial eine durchschnittliche maximale Ausdehnung bis etwa 10 mm, vorzugsweise bis etwa 6 mm, aufweist.

5. Verfahren nach Anspruch 3 oder 4, **dadurch gekennzeichnet, dass** das Pflanzenmaterial in Pulverform vorliegt.

6. Verfahren nach einem der vorangehenden Ansprüche, **dadurch gekennzeichnet, dass** das erste Extraktionsmittel ein Alkohol/Wasser-Gemisch, insbesondere ein Ethanol/Wasser-Gemisch, oder ein Aceton/Wasser-Gemisch darstellt.

7. Verfahren nach Anspruch 6, **dadurch gekennzeichnet, dass** in dem Alkohol/WasserGemisch, insbesondere Ethanol/Wasser-Gemisch, der Alkohol-Anteil im Bereich von 20 bis 80 Vol.-%, insbesondere im Bereich von 40 bis 60 Vol.-%, liegt.

8. Verfahren nach Anspruch 6 oder 7, **dadurch gekennzeichnet, dass** das Alkohol/Wasser-Gemisch, insbesondere das Ethanol/Wasser-Gemisch, im wesentlichen etwa 50 Volumenteile Alkohol, insbesondere Ethanol, und im wesentlichen etwa 50 Volumenteile Wasser enthält.

9. Verfahren nach Anspruch 6, **dadurch gekennzeichnet, dass** der Aceton-Anteil im Aceton/Wasser-Gemisch im Bereich 50 bis 90 Vol.-%, insbesondere im Bereich von 60 bis 80 Vol.-%, liegt.

10. Verfahren nach einem der vorangehenden Ansprüche, **dadurch gekennzeichnet, dass** man als Pflanzenmaterial den Krautbestandteil von *Rumex acetosa L.* verwendet.

11. Verfahren nach einem der vorangehenden Ansprüche, **dadurch gekennzeichnet, dass** man die Abtrennung des Extraktes von dem extrahierten Pflanzenmaterial mittels Filtration und/oder Zentrifugation vornimmt.

12. Verfahren nach einem der vorangehenden Ansprüche, **dadurch gekennzeichnet, dass** die Aufreinigung des Extraktes chromatographisch oder durch Ausschütteln erfolgt.

13. Verfahren nach einem der vorangehenden Ansprüche, **dadurch gekennzeichnet, dass** man das in den Schritten d), e), f) und/oder g) erhaltene Extrakt mit dem zweiten Extraktionsmittel, insbesondere in Gegenwart von Wasser, behandelt.

14. Verfahren nach Anspruch 13, **dadurch gekennzeichnet, dass** das zweite Extraktionsmittel ein unpolares organisches Lösungsmittel, insbesondere einen Kohlenwasserstoff und/oder Ethylacetat, darstellt.

15. Verfahren nach einem der vorangehenden Ansprüche, **dadurch gekennzeichnet, dass** die Extraktionsbehandlung in Schritt d) bei einer Temperatur im Bereich von 15 bis 100°C durchgeführt wird.

16. Extrakt aus Pflanzenmaterial der Art *Rumex acetosa L.,* erhalten gemäß einem Verfahren nach einem der vorangehenden Ansprüche.

17. Verwendung des Extraktes nach Anspruch 16 zur Herstellung eines Arzneimittels, insbesondere zur Prophylaxe und/oder Behandlung von Virusinfektionen oder von Virusinfektionen-bedingten Symptomen.

18. Verwendung nach Anspruch 17, **dadurch gekennzeichnet, dass** die Virusinfektion eine Infektion mit Adeno-, Herpes Simplex-, Rhino-, Influenza A-, Influenza B-, Parainfluenza-, Corona-Viren oder mit dem Respiratory syncytial- oder dem Humanes Metapneumovirus, darstellt.

19. Verwendung des Extraktes nach Anspruch 16 zur Behandlung von Virusinfektionen.

20. Verwendung nach Anspruch 19, **dadurch gekennzeichnet, dass** die Virusinfektion eine Infektion mit Adeno-, Herpes Simplex-, Rhino-, Influenza A-, Influenza B-, Parainfluenza-, Corona-Viren oder mit dem Respiratory syncytial- oder dem Humanes Metapneumovirus, darstellt.

21. Arzneimittel, Medizinprodukt, Kosmetikprodukt oder Nahrungsergänzungsmittel umfassend einen Extrakt nach Anspruch 16.
